# EUROPEAN PATENT APPLICATION

(11) **EP 4 393 469 A1**
(43) Date of publication of application: **03.07.2024**
(21) Application number: 23219821.8
(22) Date of filing: 22.12.2023
(51) Int. Cl.: A61K 8/25, A61K 8/33, A61K 8/34, A61K 8/46, A61K 8/73, A61Q 11/00, C09K 3/14

(54) **MULTICOLORED ORAL CARE COMPOSITIONS AND METHODS FOR THE SAME**

(30) Priority: 28.12.2022 CN 202211694961
(71) Applicant: Colgate-Palmolive Company, New York, NY 10022 (US)
(72) Inventor: CHEN, Dailin, Guangzhou, 510730 (CN); HUANG, Xiaoyi, Guangzhou, 510623 (CN)
(74) Representative: Sonnenhauser, Thomas Martin

(57) **Abstract**

Oral care compositions having a unique, multicolored pattern, and methods for the same are disclosed. The method for preparing an oral care composition having a unique, multicolored pattern may include forming a matrix in a base oral care composition, contacting a first colorant with a first portion of the base oral care composition, and contacting a second colorant with a second portion of the base oral care composition. The method may also include disposing the first portion of the oral care composition into a vessel via a first nozzle, disposing the second portion of the oral care composition into the vessel via a second nozzle, and forming a unique, multicolored pattern of the first and second colorants in the vessel.

## Description

### TECHNICAL FIELD

The present disclosure relates to oral care compositions and methods for the same, specifically, to oral care compositions having a unique, multicolored pattern and methods for the same.

### BACKGROUND

Aesthetic effects are known to play an important role in consumer acceptance of oral care products, such as, dentifrice or toothpaste products. These aesthetic effects allow consumers to distinguish particular products in the marketplace and to identify products having desirable, pleasing, and unique properties versus competitor products. Aesthetic effects also promote the consumer's recognition and repeated use of the product. Contrasting colored stripes or speckles are often utilized in dentifrice products to achieve these aesthetic effects. For example, these colored stripes or speckles provide an aesthetic effect that the consumer finds pleasing and promote product recognition. While incorporating colored stripes or speckles into conventional oral care products have proven to be effective for providing these aesthetic effects, the widespread use thereof has made the colored stripes and speckles commonplace; and thus, decreased the overall aesthetic effects.

What is needed, then, are improved oral care products having a unique, multicolored pattern and methods for the same.

### BRIEF SUMMARY

This summary is intended merely to introduce a simplified summary of some aspects of one or more implementations of the present disclosure. Further areas of applicability of the present disclosure will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing a method for preparing an oral care composition having a unique, multicolored pattern, the method may include: forming a matrix in a base oral care composition; contacting a first colorant with a first portion of the base oral care composition; contacting a second colorant with a second portion of the base oral care composition; disposing the first portion of the oral care composition into a vessel via a first nozzle; disposing the second portion of the oral care composition into the vessel via a second nozzle; and forming a unique, multicolored pattern of the first and second colorants in the vessel.

In at least one implementation, forming the unique, multicolored pattern of the first and second colorants in the vessel may include one or more of: controlling a rate or volume of disposing the first portion into the vessel via the first nozzle; controlling a rate or volume of disposing the second portion into the vessel via the second nozzle; controlling a rate or volume ratio between disposing the first portion into the vessel via the first nozzle and disposing the second portion into the vessel via the second nozzle; or a combination thereof.

In at least one implementation, forming the unique, multicolored pattern of the first and second colorants in the vessel may include agitating the vessel while disposing the first portion of the oral care composition into the vessel, while disposing the second portion of the oral care composition into the vessel, or while disposing both the first and second portions of the oral care composition into the vessel.

In at least one implementation, agitating the vessel may include swirling the vessel
In at least one implementation, the method may further include disposing the first portion and the second portion into the vessel at the same time.

In at least one implementation, the method may further include maintaining the unique, multicolored pattern of the first and second colorants in the vessel.

In at least one implementation, maintaining the unique, multicolored pattern of the first and second colorants in the vessel may include utilizing a pump bottle as the vessel.

In at least one implementation, maintaining the unique, multicolored pattern of the first and second colorants in the vessel may include forming the matrix in the base oral care composition, wherein the matrix is configured to inhibit or prevent the first colorant in the first portion of the oral care composition from diffusing into the second portion of the oral care composition.

In at least one implementation, forming the matrix may include contacting a combination of a thickening agent, an abrasive, an orally acceptable vehicle or one or more components thereof, or a combination thereof with one another.

In at least one implementation, the thickening agent may include a thickener silica, xanthan gum, or a combination thereof.

In at least one implementation, the abrasive may include a silica abrasive.

In at least one implementation, the orally acceptable vehicle may include a humectant and a surfactant, optionally, the humectant may include glycerin, polyethylene glycol, or a combination thereof, further optionally, the surfactant may include sodium lauryl sulfate.

In at least one implementation, forming the matrix may include contacting a combination of silica abrasive, a thickener silica, xanthan gum, glycerin, polyethylene glycol, and sodium lauryl sulfate with one another.

In at least one implementation, the thickener silica may be present in an amount of from about 2 wt% to about 8 wt%, about 3 wt% to about 7 wt%, about 4 wt% to about 6 wt%, or about 5 wt%, based on the total weight of the oral care composition.

In at least one implementation, the silica abrasive may be present in an amount of from about 6 wt% to about 10 wt%, about 7 wt% to about 9 wt%, or about 8 wt%, based on the total weight of the oral care composition.

In at least one implementation, the abrasive silica and the thickener silica may be present in a weight ratio of from about 1:1 to about 2:1, about 1.2:1 to about 1.8:1, about 1.4:1 to about 1.7:1, about 1.5:1 to about 1.6:1, or about 1.6:1.

In at least one implementation, the xanthan gum may be present in an amount of from greater than 0 wt% to about 2 wt%, about 0.2 wt% to about 1 wt%, about 0.3 wt% to about 0.7 wt%, or about 0.4 wt% to about 0.6 wt%, based on the total weight of the oral care composition.

In at least one implementation, the glycerin may be present in an amount of from about 45 wt% to about 65 wt%, about 50 wt% to about 60 wt%, about 55 wt% to about 57 wt%, or about 56 wt%, based on the total weight of the oral care composition.

In at least one implementation, the polyethylene glycol may be present in an amount of from about 1 wt% to about 5 wt%, about 2 wt% to about 4 wt%, or about 3 wt%, based on the total weight of the oral care composition.

In at least one implementation, the sodium lauryl sulfate may be present in an amount of from greater than 0 wt% to about 4 wt%, about 1 wt% to about 3 wt%, or about 2 wt%, based on the total weight of the oral care composition.

In at least one implementation, the method may further include maintaining the unique, multicolored pattern of the first and second colorants in the vessel while dispensing the oral care composition from the vessel.

The foregoing and/or other aspects and utilities embodied in the present disclosure may be achieved by providing a method for enhancing aesthetic effects of an oral care composition. The method may include the method for preparing the oral care composition disclosed herein.

The foregoing and/or other aspects and utilities embodied in the present disclosure may be achieved by providing an oral care composition a base oral care composition comprising a matrix, the matrix being configured to prevent or inhibit a first colorant in a first portion of the base oral care composition from diffusing into or mixing with a second portion of the base oral care composition, the matrix comprising a thickening agent, an abrasive, an orally acceptable vehicle or a component thereof, or a combination thereof.

In at least one implementation, the thickening agent may include a thickener silica, xanthan gum, or a combination thereof; the abrasive may include a silica abrasive; the orally acceptable vehicle may include a humectant and a surfactant, optionally, the humectant may include glycerin, polyethylene glycol, or a combination thereof, further optionally, the surfactant may include sodium lauryl sulfate.

In at least one implementation, the matrix may include a combination of the silica abrasive, the thickener silica, xanthan gum, glycerin, polyethylene glycol, and sodium lauryl sulfate.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure.

### DETAILED DESCRIPTION

The following description of various typical aspect(s) is merely exemplary in nature and is in no way intended to limit the disclosure, its application, or uses.

As used throughout this disclosure, ranges are used as shorthand for describing each and every value that is within the range. It should be appreciated and understood that the description in a range format is merely for convenience and brevity and should not be construed as an inflexible limitation on the scope of any embodiments or implementations disclosed herein. Accordingly, the disclosed range should be construed to have specifically disclosed all the possible subranges as well as individual numerical values within that range. As such, any value within the range may be selected as the terminus of the range. For example, description of a range such as from 1 to 5 should be considered to have specifically disclosed subranges such as from 1.5 to 3, from 1 to 4.5, from 2 to 5, from 3.1 to 5, etc., as well as individual numbers within that range, for example, 1, 2, 3, 3.2, 4, 5, etc. This applies regardless of the breadth of the range.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

As used herein, "free" or "substantially free" of a material may refer to a composition, component, or phase where the material is present in an amount of less than 10.0 wt%, less than 5.0 wt%, less than 3.0 wt%, less than 1.0 wt%, less than 0.1 wt%, less than 0.05 wt%, less than 0.01 wt%, less than 0.005 wt%, or less than 0.0001 wt% based on a total weight of the composition, component, or phase.

All references cited herein are hereby incorporated by reference in their entireties. In the event of a conflict in a definition in the present disclosure and that of a cited reference, the present disclosure controls.

### COMPOSITIONS

Compositions disclosed herein may be or include an oral care product or an oral care composition thereof. For example, the composition may be an oral care product including the oral care composition, or the oral care composition thereof. Illustrative oral care products or oral care compositions may be or include, but are not limited to, a toothpaste (dentifrice), a prophylactic paste, a tooth gel (e.g., a whitening gel), or the like. In an exemplary implementation, the oral care products or the oral care composition disclosed herein is a dentifrice or toothpaste. For example, the oral care composition disclosed herein may be a toothpaste having a multicolored pattern, such as a unique, multicolored pattern that maintains the pattern in a vessel (e.g., bottle). As further disclosed herein, the oral care composition may maintain the unique, multicolored pattern in the vessel even while dispensing the composition from the vessel (e.g., pump bottle).

The oral care composition may include an orally acceptable vehicle, one or more abrasives or abrasive compounds, one or more thickening agents, a plurality of colorants, or a combination thereof. The oral care composition includes at least a first portion and a second portion, wherein the first portion includes a first colorant, and the second portion includes a second colorant. It should be appreciated that the oral care composition may also include a third or additional portions, each of which may include a different respective colorant. Each of the portions and the respective colorants thereof may provide or facilitate the formation of oral care compositions having or exhibiting a unique, multicolored pattern. As used herein, the term or expression "multicolor" or "multicolored" may refer to a substance or composition having a plurality of portions, each of the plurality of portions having a different color, different shades of the same color, different hues of the same color, or the like.

In at least one implementation, the oral care composition may have or exhibit a multicolored pattern of the colorants, wherein the respective colorants are at least substantially non-bleeding, non-diffusing, non-blurring, or otherwise non-mixing. For example, the colorant of the first portion of the oral care composition exhibits minimal, substantially none, or no bleeding, blurring, or otherwise mixing with the colorant of the second or another portion of the oral care composition. In another example, the first colorant of the first portion of the oral care composition exhibits minimal, substantially none, or no diffusion into the second portion of the oral care composition including the second colorant. Without being bound by theory, it is believed that the suppression of bleeding, diffusion, or mixing of the first colorant in the first portion into the second colorant of the second portion may be provided, at least in part, by the combination of ingredients/components of the composition. In at least one implementation, the suppression of bleeding, diffusion, or mixing of the first colorant in the first portion into the second colorant of the second portion may be provided by the combination of the thickening agents, the abrasives, the orally acceptable vehicle and/or a component thereof, or a combination thereof. For example, the suppression of bleeding, diffusion, or mixing of the first colorant in the first portion into the second colorant of the second portion may be provided by a matrix formed by a combination of the thickening agents, the abrasives, the orally acceptable vehicle and/or a component thereof, or a combination thereof. The matrix may prevent or inhibit the respective colorant in one portion of the oral care composition from diffusing into or mixing with another portion of the oral care composition. In a preferred implementation, the matrix is formed from a combination of the thickening agents, the abrasives, the orally acceptable vehicle and/or a component thereof, or a combination thereof. For example, the matrix may be formed from a combination of abrasive silica, one or more thickening agents, including but not limited to, a silica thickener or thickener silica, xanthan gum, or a combination thereof, one or more humectants of the orally acceptable vehicle, including but not limited to, glycerin, polyethylene glycol, or a combination thereof, one or more surfactants of the orally acceptable vehicle, including but not limited to, sodium lauryl sulfate. In an exemplary implementation, the matrix is formed from a combination of abrasive silica, a thickener silica, xanthan gum, glycerin, polyethylene glycol, and sodium lauryl sulfate, each in an amount and/or ratio effective to form the matrix. The abrasive silica may be present in an amount of from about 6 wt% to about 10 wt%, about 7 wt% to about 9 wt%, or about 8 wt%, based on the total weight of the oral care composition. The thickener silica may be present in an amount of from about 2 wt% to about 8 wt%, about 3 wt% to about 7 wt%, about 4 wt% to about 6 wt%, or about 5 wt%. A weight ratio of the abrasive silica to the thickener silica may be from about 1:1 to about 2:1, about 1.2:1 to about 1.8:1, about 1.4:1 to about 1.7:1, about 1.5:1 to about 1.6:1, or about 1.6:1. The xanthan gum may be present in an amount of from greater than 0 wt% to about 2 wt%, about 0.1 wt% to about 1.5 wt%, about 0.2 wt% to about 1 wt%, about 0.3 wt% to about 0.7 wt%, or about 0.4 wt% to about 0.6 wt%, or about 0.4 wt% to about 0.5 wt%. The glycerin may be present in an amount of from about 45 wt% to about 65 wt%, about 50 wt% to about 60 wt%, about 55 wt% to about 57 wt%, or about 56 wt%. The polyethylene glycol may be present in an amount of from about 1 wt% to about 5 wt%, about 2 wt% to about 4 wt%, or about 3 wt%. The sodium lauryl sulfate may be present in an amount of from greater than 0 wt% to about 4 wt%, about 1 wt% to about 3 wt%, or about 2 wt%.

As disclosed herein, the multicolored oral care composition may be provided by the colorant of the first portion of the oral care composition and the colorant of the second portion of the oral care composition. The first portion and the second portion of the oral care composition are in contact with one another, but are arranged or disposed in a random, unique pattern. As such, the first portion and the second portion of the oral care compositions are not disposed in parallel layers. For example, the first and second portions of the oral care composition are not disposed in ordered horizontal or vertical layers.

The first and second portions of the oral care composition may include the same or substantially the same composition (e.g., base composition) except for the respective colorants thereof. For example, the first and second portions of the oral care composition may include the same or substantially the same orally acceptable vehicle, abrasives or abrasive compounds, thickening agents, or a combination thereof, but may further include different colorants.

The oral care products and oral care compositions disclosed herein may be disposed in any dispensing means. The dispensing means may be at least partially transparent such that the unique, multicolored pattern of the oral care composition is visible to the consumer. Illustrative dispensing means may be or include, but are not limited to, a collapsible tube, a pump bottle, or any other vessel known in the art for dispensing oral care compositions, especially dentifrice compositions. In a preferred implementation, the oral care products or the oral care compositions utilize a pump bottle capable of or configured to dispense the oral care product or the oral care composition by drawing the oral care product or the oral care composition from the bottom of the bottle. It should be appreciated that the matrix formed in the oral care composition allows the oral care composition to maintain its unique multicolored pattern as the pump bottle dispenses the oral care composition. It should be further appreciated that a rigidity of the pump bottle may allow the composition to maintain the unique multicolored pattern. For example, utilizing a collapsible tube would require the tube to be compressed to dispense the oral care composition, and the compression of the tube may disrupt or destroy the unique multicolored pattern formed during manufacture.

The oral care product or the oral care composition thereof may include one or more abrasives or abrasive compounds. As used herein, the term "abrasive" may refer to materials commonly referred to as "polishing agents." The one or more abrasives may be mixed, combined, dispersed, suspended, or otherwise contacted with the orally acceptable vehicle. In at least one implementation, the oral care product or the whitening composition thereof includes a single abrasive. In another implementation, the oral care product or the whitening composition thereof includes a mixture or combination of at least two abrasives. Illustrative abrasives may be or include, but are not limited to, metaphosphate compounds, phosphate salts (e.g., insoluble phosphate salts), such as sodium metaphosphate, potassium metaphosphate, calcium pyrophosphate, magnesium orthophosphate, trimagnesium orthophosphate, tricalcium phosphate, dicalcium phosphate dihydrate, anhydrous dicalcium phosphate, calcium carbonate, magnesium carbonate, hydrated alumina, abrasive silicas, silica, silicates, zirconium silicate, aluminum silicate, calcined aluminum silicate, polymethyl methacrylate, or the like, or a combination thereof. In an exemplary implementation, the abrasives include at least the silica abrasives, such as those available under the trade designation ZEODENT^{®} 115, which are commercially available from Evonic Industries, AG of Essen Germany.

The one or more of the abrasives in the abrasive system may have a pellicle cleaning ratio (PCR) greater than or equal to 80, greater than or equal to 82, greater than or equal to 84, greater than or equal to 86, greater than or equal to 88, greater than or equal to 90, greater than or equal to 92, greater than or equal to 94, greater than or equal to 96, greater than or equal to 98, greater than or equal to 100, greater than or equal to 102, greater than or equal to 104, greater than or equal to 106, greater than or equal to 108, greater than or equal to 110, greater than or equal to 112, or greater.

The amount or concentration of the one or more abrasives present in the oral care product or the oral care composition thereof may vary widely. In at least one implementation, the amount or concentration of the abrasives may be from greater than 0 wt% to about 30 wt%, based on a total weight of the oral care product or the composition thereof. For example, the amount of the abrasives present in the oral care composition may be from greater than 0 wt%, about 1 wt%, about 2 wt%, about 3 wt%, about 4 wt%, about 5 wt%, about 6 wt%, about 7 wt%, or about 8 wt% to about 10 wt%, about 11 wt%, about 12 wt%, about 13 wt%, about 14 wt%, about 15 wt%, about 16 wt%, about 17 wt%, about 18 wt%, about 20 wt%, or about 30 wt%. In another example, the amount of the abrasives present in the oral care composition may be from greater than 0 wt% to about 20 wt%, about 2 wt% to about 18 wt%, about 5 wt% to about 15 wt%, about 7 wt% to about 9 wt%, or about 8 wt%, based on the total weight of the oral care product or the oral care composition thereof. In a preferred implementation, the amount of the abrasives present in the oral care composition may be from about 5 wt% to about 11 wt%, preferably about 6 wt% to about 10 wt%, or more preferably about 8 wt%, based on a total weight of the oral care product or the composition thereof.

The oral care product or the oral care composition thereof may include one or more thickeners. The one or more thickeners may be any orally acceptable thickener or thickening agent configured to control the viscosity of the oral care product or the whitening composition thereof. Illustrative thickeners may be or include, but are not limited to, colloidal silica, fumed silica, thickening silica, polyvinylpyrrolidone (PVP), a cross-linked polyvinylpyrrolidone (PVP) polymer, cross-linked polyvinylpyrrolidone (PVP), or the like, or a combination thereof. In at least one implementation, the thickeners include at least thickening silica. Illustrative thickeners may also be or include, POLYPLASDONE^{®} XL 10F, which is commercially available from Ashland Inc. of Covington, KY. Illustrative thickeners may further be or include, but are not limited to, carbomers (e.g., carboxyvinyl polymers), carrageenans (e.g., Irish moss, carrageenan, iota-carrageenan, etc.), high molecular weight polyethylene glycols (e.g., CARBOWAX^{®}, which is commercially available from The Dow Chemical Company of Midland, MI), cellulosic polymers, hydroxyethylcellulose, carboxymethylcellulose, and salts thereof (e.g., CMC sodium), natural gums (e.g., karaya, xanthan, gum arabic, and tragacanth), colloidal magnesium aluminum silicate, or the like, or a combination thereof. In at least one implementation, the one or more thickeners may be or include, but are not limited to, an organic polymer. Illustrative organic polymers may be or include, but are not limited to, hydrophilic polymers, such as carbomers, such as carboxymethylene polymers, such as acrylic acid polymers, and acrylic acid copolymers. carboxypolymethylene is a slightly acidic vinyl polymer with active carboxyl groups. In a typical embodiment, the thickening system includes a carboxypolymethylene, such as CARBOPOL^{®} 974 and/or 980, which are commercially available from Noveon, Inc. of Cleveland, OH. In at least one implementation, the one or more thickeners includes a thickener silica, xanthan gum, or a combination thereof. For example, the one or more thickeners includes a combination of a thickener silica and xanthan gum.

The amount or concentration of any one or more of the thickeners present in the oral care product or the oral care composition thereof may vary widely. The amount of any one or more of the thickeners present in the oral care product or the oral care composition thereof may be from about 0.1 wt% to about 50 wt%, based on the total weight of the oral care product or the oral care composition thereof. For example, the amount of any one or more of the thickeners present may be from about 0.1 wt%, about 0.2 wt%, about 0.3 wt%, about 0.4 wt%, about 0.5 wt%, about 0.6 wt%, about 0.8 wt%, about 1 wt%, about 3 wt%, about 4 wt%, or about 5 wt% to about 5.5 wt%, about 6 wt%, about 8 wt%, about 10 wt%, about 30 wt%, or about 50 wt%. In at least one embodiment, the one or more thickeners includes a thickener silica, xanthan gum, or a combination thereof. For example, the thickeners include a combination of a thickener silica and xanthan gum. The thickener silica may be present in an amount of from about 2 wt% to about 8 wt%, about 3 wt% to about 7 wt%, about 4 wt% to about 6 wt%, or about 5 wt%. The xanthan gum may be present in an amount of from about 0.1 wt% to about 1 wt%, about 0.2 wt% to about 0.8 wt%, about 0.3 wt% to about 0.7 wt%, about 0.4 wt% to about 0.6 wt%, or about 0.5 wt%, based on the total weight of the oral care composition.

The plurality of colorants may be or include, but are not limited to, pigments, dyes, lakes, or the like, or a combination thereof. The plurality of colorants may be or include oil-soluble pigments or water immiscible pigments, dyes, water-soluble dyes and lakes, including natural or synthetic dyes, or a combination thereof. In an exemplary implementation, the plurality of colorants includes at least one oil-soluble or water immiscible colorant. It should be appreciated that any orally acceptable colorant may be utilized, including, but not limited to, FD&C dyes and pigments, talc, mica, magnesium carbonate, magnesium silicate, silica, titanium dioxide, zinc oxide, red, yellow, brown and black iron oxides, ferric ammonium ferrocyanide, manganese violet, ultramarine, titanated mica, bismuth oxychloride, or the like, or a combination thereof.

Illustrative colorants may be or include, but are not limited to, those listed in Title 21 of the U.S. Code of Federal Regulations, Section 74, including for example FD&C Blue #1 and FD&C Yellow #10, Eyeshadow Blue KO, Colour Index 77 510, EG-No., Blue 15 (C-Blue 17), or mixtures of one or more water-insoluble dyes and one or more water-soluble dyes, for example Eyeshadow Blue KO and Lemon Yellow ZN 3, in which case green hues are obtained, lakes, such as lakes certified by the Color Certification Laboratory of the Food and Drug, Administration of the Health, Education and Welfare Department of the United States Government, for example, F.D. & C. Blue No. 1 Lake, F.D. & C. Blue No. 2 Lake, F.D. & C. Red No. 3 Lake, F.D. & C. Yellow No. 5 Lake and F.D. & C. Yellow No. 6 Lake, or the like, or a combination thereof.

Any one or more of the plurality of colorants may be present in an amount of from about 0.0005 wt% or more to about 30 wt% or more, based on the total weight of the oral care composition. For example, any one or more of the colorants may be present in an amount of from about 0.0005 wt%, about 0.001 wt%, about 0.01 wt%, about 0.1 wt%, or more to about 5 wt%, about 20%, about 30 wt%, or more, based on the total weight of the oral care composition

The orally acceptable vehicle may include one or more humectants. Illustrative humectants may be or include, but are not limited to, glycerin, propylene glycol, polyethylene glycol (PEG) (e.g., PEG 400, PEG 600, etc.), sorbitol, xylitol, or the like, or a mixture or combination thereof. In a at least one implementation, the humectants of the orally acceptable vehicle include glycerin, polyethylene glycol, or a combination thereof. In an exemplary implementation, the humectants of the orally acceptable vehicle include a combination of glycerin and polyethylene glycol.

Any one or more of the humectants may be present in the oral care composition an amount of from about 1 wt% to about 70 wt%, based on a total weight of the oral care product or the whitening composition thereof. For example, any one or more humectants may be present in an amount of from about 1 wt%, about 2 wt%, about 3 wt%, about 5 wt%, or about 10 wt% to about 20 wt%, about 30 wt%, about 40 wt%, about 45 wt%, about 50 wt%, about 55 wt%, about 60 wt%, or about 70 wt%, based on a total weight of the oral care composition. In an exemplary implementation, the one or more humectants include glycerin and PEG, the glycerin may be present in an amount of from about 40 wt% to about 70 wt%, about 45 wt% to about 65 wt%, about 50 wt% to about 60 wt%, about 55 wt% or about 56 wt%, based on the total weight of the oral care composition, and the PEG may be present in an amount of from about 1 wt%, about 2 wt%, about 2.5 wt%, or about 3 wt% to about 3.5 wt%, about 4 wt%, about 5 wt%, or about 6 wt%, based on the total weight of the oral care composition.

The orally acceptable vehicle may include an orally acceptable solvent. Illustrative solvents may include, but are not limited to, one or more of ethanol, phenoxyethanol, isopropanol, water, cyclohexane, methyl glycol acetate, benzyl alcohol, or the like, or a mixture or combination thereof. In an exemplary implementation, the orally acceptable solvent includes water, preferably demineralized water. The orally acceptable solvent may be present in an amount of from about 10 wt% to about 30 wt%, based on the total weight of the oral care composition. For example, the orally acceptable solvent may be present in an amount of from about 10 wt%, about 12 wt%, about 16 wt%, or about 18 wt% to about 20 wt%, about 24 wt%, about 26 wt%, or about 30 wt%, based on the total weight of the oral care composition.

The orally acceptable vehicle may include one or more surfactants. The one or more surfactants may be or include, but are not limited to, one or more anionic surfactants, one or more cationic surfactants, one or more zwitterionic surfactants, one or more nonionic surfactants, or a mixture or combination thereof.

Illustrative anionic surfactants may be or include, but are not limited to, one or more C6-C18 fatty acid glutamate salts, water-soluble salts of C8-20 alkyl sulfates, sulfonated monoglycerides of C8-20 fatty acids, sarcosinates, taurates, sodium lauryl sulfate, sodium cocoyl monoglyceride sulfonate, sodium lauryl sarcosinate, sodium lauryl isoethionate, sodium laureth carboxylate, sodium lauroyl methyl taurate, sodium dodecyl benzenesulfonate, water-soluble salts of higher fatty acid monoglyceride monosulfates, such as the sodium salt of the monosulfated monoglyceride of hydrogenated coconut oil fatty acids such as a sodium N-methyl-N-alkyl taurate, sodium N-methyl-N-cocoyl taurate or sodium methyl cocoyl taurate, sodium cocoyl methyl taurate, sodium lauroyl methyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOSO₃X, wherein m is 6-16, e.g., 10, n is 1-6, e.g. 2, 3 or 4, and X is Na, for example sodium laureth-2 sulfate (CH₃(CH₂)₁₀CH₂(OCH₂CH₂)₂OSO₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide), sodium lauryl sarcosinate, or the like, or any mixture or combination thereof. As used herein, "higher alkyl" may refer to a C₆-C₃₀ alkyl. In at least one implementation, the anionic surfactant may include one or more C6-C18 fatty acid glutamate salts or amino acid surfactants, such as a cocoyl glutamate salt, particularly sodium cocoyl glutamate. In an exemplary implementation, the one or more surfactants includes one or more anionic surfactants, and the anionic surfactants include sodium lauryl sulfate.

The amphoteric and zwitterionic surfactants may be or include, but are not limited to, derivatives of C₈₋₂₀ aliphatic secondary and tertiary amines having an anionic group such as carboxylate, sulfate, sulfonate, phosphate or phosphonate. Illustrative amphoteric and zwitterionic surfactants may include, but are not limited to, sultaines and betaines, such as cocamidopropyl betaine (CAPB), derivatives of aliphatic secondary and tertiary amines in which the aliphatic radical can be a straight or branched chain and wherein one of the aliphatic substituents contains about 8-18 carbon atoms and one contains an anionic water-solubilizing group, such as carboxylate, sulfonate, sulfate, phosphate or phosphonate, or the like, or a combination thereof.

Illustrative nonionic surfactants may be or include, but are not limited to, one or more alkyl polyglucosides, such as one or more C6-C18 alkyl polyglucosides, or the like. The C6-C18 alkyl polyglucosides may include one or more short chain alkyl polyglucosides, one or more long chain alkyl polyglucosides, or any combination thereof. As used herein, the term or expression "short chain alkyl polyglucosides" may refer to alkyl polyglucosides having a chain length of from about 6 to about 10 carbon atoms. As used herein, the term or expression "long chain alkyl polyglucosides" may refer to alkyl polyglucosides having a chain length of from about 11 to about 18 carbon atoms. It should be appreciated that the alkyl polyglucosides may include a hydrophobic fatty alcohol portion and a hydrophilic glucoside portion. In a preferred implementation, the nonionic surfactants include at least a short chain alkyl polyglucoside, such as a C6-C10 alkyl polyglucoside or a C8-C10 alkyl polyglucoside (CAS 68515-73-1), which is commercially available as APG 810. In at least one implementation, the oral care composition is free or substantially free of long chain alkyl polyglucosides. Additional illustrative nonionic surfactants may be or include, but are not limited to, one or more of octoxynol (e.g., Macrogol tetramethylbutylphenyl ether, octylphenoxy polyethoxyethanol, or polyoxyethylene octylphenyl ether), such as octoxynol 1, 3, 5, 8, 9, 10, 12, 13, 16, 30, 40, 70, wherein the number indicates the number of repeating oxyethylene units, or other octoxynols that include different numbers of repeating units of oxyethylene in the side chain, sorbitan esters (e.g., sorbitan monooleate and sorbitan monostearate, etc.) commonly known by their trade names SPAN^{®} 80 and SPAN^{®} 60), polysorbates (e.g., polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of TWEEN^{®} 80, TWEEN^{®} 60, Tween^{®} 20), poloxamers (synthetic block polymers of ethylene oxide and propylene oxide, such as those commonly known by their trade names of PLURONIC^{®}; e.g., PLURONIC^{®} F127 or PLURONIC^{®} F108), or poloxamines (synthetic block polymers of ethylene oxide and propylene oxide attached to ethylene diamine, such as those commonly known by their trade names of TETRONIC^{®}; e.g., TETRONIC^{®} 1508 or TETRONIC^{®} 908, etc.), other nonionic surfactants such as BRIJ^{®} (polyoxyethylene alkyl ether having a formula of CH₃-(CH₂)₁₀₋₁₆-(O-C₂H₄)₁₋₂₅-OH), MYRJ^{®} (stearic acid esterified with polyoxyethylene having 40-100 repeating oxyethylene units), long chain fatty alcohols (e.g., oleyl alcohol, stearyl alcohol, myristyl alcohol, docosahexaenoyl alcohol, etc.) with carbon chains having about 12 or more carbon atoms (e.g., such as from about 12 to about 24 carbon atoms), or the like, or any mixture or combination thereof. Additional nonionic surfactants may be or include, but are not limited to, polyethylene oxide condensates of alkyl phenols, products derived from the condensation of ethylene oxide with the reaction product of propylene oxide and ethylenediamine, ethylene oxide condensates of aliphatic alcohols, long chain tertiary amine oxides, long chain tertiary phosphine oxides, long chain dialkyl sulfoxides, or the like, or combinations thereof. In at least one implementation, the nonionic surfactants may be broadly defined as compounds produced by the condensation of alkylene oxide groups (hydrophilic in nature) with an organic hydrophobic compound, which may be aliphatic or alkylaromatic in nature.

The amount of any one or more of the surfactants present in the oral care composition may vary widely. For example, the amount of any one or more of the surfactants present in the oral care composition may be greater than 0.0 wt% or 0.01 wt% and less than or equal to 10.0 wt%, based on a total weight of the oral care composition. For example, the amount of any one or more of the surfactants present in the oral care composition may be from greater than 0 wt%, about 0.01 wt%, about 0.02 wt%, about 0.03 wt%, about 0.04 wt%, about 0.05 wt%, or about 0.06 wt% to about 1 wt%, about 2 wt%, about 3 wt%, about 4 wt%, about 5 wt%, about 8 wt%, or about 10 wt%, based on a total weight of the oral care composition. In an exemplary implementation, each of the one or more of the surfactants may, separately and independently, be present in the oral care composition in an amount of from about 0.01 wt% to about 3 wt%, about 0.02 wt% to about 1 wt%, about 0.03 wt% to about 0.5 wt%, about 0.04 wt% to about 0.1 wt%, or about 0.05 wt%, based on a total weight of the oral care composition. In an exemplary implementation, each of the one or more of the surfactants may, separately and independently, be present in the oral care composition in an amount of from about 1 wt% to about 10 wt%, about 2 wt% to about 8 wt%, about 3 wt% to about 7 wt%, about 4 wt% to about 6 wt%, or about 5 wt%, based on a total weight of the oral care composition.

The orally acceptable vehicle may make up the balance of the oral care product or the oral care composition thereof. The orally acceptable vehicle may be present in an amount of at least 60 wt%, at least 62 wt%, at least 64 wt%, at least 66 wt%, at least 68 wt%, at least 70 wt%, at least 72 wt%, at least 74 wt%, at least 76 wt%, at least 78 wt%, at least 80 wt%, at least 82 wt%, at least 84 wt%, at least 86 wt%, at least 88 wt%, at least 90 wt%, at least 92 wt%, at least 94 wt%, at least 96 wt%, at least 98 wt%, at least 99 wt%, or more, based on a total weight of the oral care product or the oral care composition thereof.

The oral care composition or the orally acceptable vehicle thereof may also include one or more flavoring agents. Illustrative flavoring agents that may be or include, but are not limited to, essential oils and various flavoring aldehydes, esters, alcohols, and similar materials, as well as sweeteners such as sodium saccharin and/or sorbitol, peppermint flavor (e.g., OPTAMINT^{®}), FREEZESTORM^{™}, which is commercially available from Firmenich of Geneva, Switzerland, or the like, or a combination thereof. Illustrative essential oils may include, but are not limited to, oils of spearmint, peppermint, wintergreen, sassafras, clove, sage, eucalyptus, marjoram, cinnamon, lemon, lime, grapefruit, and orange. Also useful are chemicals such as menthol, carvone, anethole, or the like, or a mixture or combination thereof.

The amount or concentration of the one or more flavoring agents present in the oral care product or the oral care composition thereof may vary widely. For example, the amount of the flavoring agents present may be from about 0.01 wt% to about 10 wt%, based on a total weight of the oral care product or the oral care composition thereof. For example, the amount of the one or more flavoring agents present may be from about 0.01 wt%, about 1 wt%, about 2 wt%, about 3 wt%, about 4 wt%, about 5 wt%, or about 6 wt% to about 7 wt%, about 8 wt%, about 9 wt%, or about 10 wt%, based on the total weight of the oral care composition.

It should be appreciated by one having ordinary skill in the art, that the oral care products and/or the oral care compositions thereof may include other additional ingredients/components. For example, the oral care products and/or the oral care compositions thereof may include, one or more antibacterial agents, fluoride ion sources (e.g., sodium fluoride), anticalculus agents, buffers, sources of peroxide (e.g., hydrogen peroxide), alkali metal bicarbonate salts, antimicrobial agents, additional water, titanium dioxide, cooling agents, desensitizing agents, additional viscosity modifiers, diluents, surface active agents (e.g., emulsifiers, foam modulators, etc.), pH modifying agents (e.g., acids and bases), mouth feel agents, sweetening agents, preservatives, or the like, or any mixture or combination thereof. It should further be appreciated by one having ordinary skill in the art that while general attributes of each of the above categories of materials may differ, there may be some common attributes and any given material may serve multiple purposes within two or more of such categories of materials.

In at least one implementation, the oral care product or the oral care composition thereof may exclude any one or more of the aforementioned materials disclosed.

All ingredients for use in the compositions described herein are orally acceptable. As used herein, "orally acceptable" may refer to any ingredient that is present in a composition as described in an amount and form that does not render the composition unsafe for use in the oral cavity.

### METHODS

The present disclosure may provide methods for preparing oral care compositions (e.g., toothpastes) having a unique, multicolored pattern that maintains the pattern in a vessel (e.g., pump bottle). For example, the oral care composition may maintain the multicolored pattern formed in the vessel (e.g., during manufacture) and maintain the multicolored pattern while being dispensed from the vessel. The method may include combining an orally acceptable vehicle, one or more abrasives or abrasive compounds, one or more thickening agents, or a combination thereof to prepare a base oral care composition. The method may also include combining, mixing, or otherwise contacting a first colorant with a first portion of the base oral care composition. The method may also include combining, mixing, or otherwise contacting a second colorant with a second portion of the base oral care composition. The method may also include disposing the first portion of the oral care composition into a vessel via a first nozzle. The method may also include disposing the second portion of the oral care composition into the vessel via a second nozzle. The first and second portions may be disposed into the vessel at the same or substantially the same time. The method may include disposing the first portion into the vessel at a first rate and disposing the second portion into the vessel at a second rate, wherein the first rate and the second rate may be the same or different. The method may also include varying a ratio (e.g., volume, flow, speed, etc.) between the dispensing of the first and second portions into the vessel. The method may also include agitating the vessel while disposing the first portion and/or the second portion of the oral care composition into the vessel. Agitating the vessel may include swirling, whirling, spinning, mixing, vibrating, or the like, or a combination thereof. The speed or rate of agitation may be varied. It should be appreciated that the general appearance or aesthetic effect of the multicolored pattern may be determined, at least in part, by the rate of dispensing the first portion into the vessel, the rate of dispensing the second portion into the vessel, the rate of agitating the vessel while dispensing the first and second portions into the vessel, or a combination thereof. It should further be appreciated, as demonstrated in the Example disclosed herein, that the general appearance or aesthetic effect of the multicolored pattern may be provided consistently by controlling or maintaining the rate of dispensing the first portion into the vessel, the rate of dispensing the second portion into the vessel, the rate of agitating the vessel while dispensing the first and second portions into the vessel, or a combination thereof. The method may include forming a matrix in the oral care composition, wherein the matrix is capable of or configured to prevent or inhibit the first colorant in the first portion of the oral care composition from diffusing into the second portion of the oral care composition. Forming the matrix may include combining or otherwise contacting a combination of abrasive silica, one or more thickening agents, including but not limited to, a thickener silica, xanthan gum, or a combination thereof, one or more humectants of the orally acceptable vehicle, including but not limited to, glycerin, polyethylene glycol, or a combination thereof, one or more surfactants of the orally acceptable vehicle, including but not limited to, sodium lauryl sulfate, with one another. For example, forming the matrix may include contacting abrasive silica, a thickener silica, xanthan gum, glycerin, polyethylene glycol, sodium lauryl sulfate, or a combination thereof, with one another. Particularly, forming the matrix may include mixing, combining, or otherwise contacting a combination of abrasive silica, a thickener silica, xanthan gum, glycerin, polyethylene glycol, and sodium lauryl sulfate. The abrasive silica may be present in an amount of from about 6 wt% to about 10 wt%, about 7 wt% to about 9 wt%, or about 8 wt%, based on the total weight of the oral care composition. The thickener silica may be present in an amount of from about 2 wt% to about 8 wt%, about 3 wt% to about 7 wt%, about 4 wt% to about 6 wt%, or about 5 wt%. A weight ratio of the abrasive silica to the thickener silica may be from about 1:1 to about 2:1, about 1.2:1 to about 1.8:1, about 1.4:1 to about 1.7:1, about 1.5:1 to about 1.6:1, or about 1.6:1. The xanthan gum may be present in an amount of from greater than 0 wt% to about 2 wt%, about 0.1 wt% to about 1.5 wt%, about 0.2 wt% to about 1 wt%, about 0.3 wt% to about 0.7 wt%, or about 0.4 wt% to about 0.6 wt%, or about 0.4 wt% to about 0.5 wt%. The glycerin may be present in an amount of from about 45 wt% to about 65 wt%, about 50 wt% to about 60 wt%, about 55 wt% to about 57 wt%, or about 56 wt%. The polyethylene glycol may be present in an amount of from about 1 wt% to about 5 wt%, about 2 wt% to about 4 wt%, or about 3 wt%. The sodium lauryl sulfate may be present in an amount of from greater than 0 wt% to about 4 wt%, about 1 wt% to about 3 wt%, or about 2 wt%. The method may also include entraining the first colorant in the matrix of the first portion of the oral care composition. The method may also include entraining the second colorant in the matrix of the second portion of the oral care composition. The method may further include maintaining the first colorant and the second colorant in the first portion and the second portion, respectively, with the matrix of the oral care composition. The first portion of the oral care composition and the second portion of the oral care composition may be maintained separate from one another outside of the vessel.

The present disclosure may provide a method for communicating or informing a consumer of one or more properties of an oral care product or an oral care composition thereof. The method may include preparing the oral care compositions having a unique, multicolored pattern disclosed herein, and disposing the oral care compositions in a vessel (e.g., bottle).

The present disclosure may also provide a method for enhancing aesthetic effects of an oral care product or an oral care composition thereof to a consumer. The method may include preparing the oral care compositions having a unique, multicolored pattern disclosed herein, and disposing the oral care compositions in a vessel (e.g., bottle).

The present disclosure may provide a method for suppressing or preventing the diffusion or blending of a first color in a first portion of an oral care composition into or with a second color in a second portion of the oral care composition. The method may include forming a matrix in the oral care composition, wherein the matrix is capable of or configured to prevent or inhibit the first colorant in the first portion of the oral care composition from diffusing into the second portion of the oral care composition. Forming the matrix may include combining or otherwise contacting a combination of abrasive silica, one or more thickening agents, including but not limited to, a thickener silica, xanthan gum, or a combination thereof, one or more humectants of the orally acceptable vehicle, including but not limited to, glycerin, polyethylene glycol, or a combination thereof, one or more surfactants of the orally acceptable vehicle, including but not limited to, sodium lauryl sulfate, with one another. For example, forming the matrix may include contacting abrasive silica, a thickener silica, xanthan gum, glycerin, polyethylene glycol, sodium lauryl sulfate, or a combination thereof, with one another. Particularly, forming the matrix may include mixing, combining, or otherwise contacting a combination of abrasive silica, a thickener silica, xanthan gum, glycerin, polyethylene glycol, and sodium lauryl sulfate. The abrasive silica may be present in an amount of from about 6 wt% to about 10 wt%, about 7 wt% to about 9 wt%, or about 8 wt%, based on the total weight of the oral care composition. The thickener silica may be present in an amount of from about 2 wt% to about 8 wt%, about 3 wt% to about 7 wt%, about 4 wt% to about 6 wt%, or about 5 wt%. A weight ratio of the abrasive silica to the thickener silica may be from about 1:1 to about 2:1, about 1.2:1 to about 1.8:1, about 1.4:1 to about 1.7:1, about 1.5:1 to about 1.6:1, or about 1.6:1. The xanthan gum may be present in an amount of from greater than 0 wt% to about 2 wt%, about 0.1 wt% to about 1.5 wt%, about 0.2 wt% to about 1 wt%, about 0.3 wt% to about 0.7 wt%, or about 0.4 wt% to about 0.6 wt%, or about 0.4 wt% to about 0.5 wt%. The glycerin may be present in an amount of from about 45 wt% to about 65 wt%, about 50 wt% to about 60 wt%, about 55 wt% to about 57 wt%, or about 56 wt%. The polyethylene glycol may be present in an amount of from about 1 wt% to about 5 wt%, about 2 wt% to about 4 wt%, or about 3 wt%. The sodium lauryl sulfate may be present in an amount of from greater than 0 wt% to about 4 wt%, about 1 wt% to about 3 wt%, or about 2 wt%. The method may also include entraining the first colorant in the matrix of the first portion of the oral care composition. The method may also include entraining the second colorant in the matrix of the second portion of the oral care composition. The method may further include maintaining the first colorant and the second colorant in the first portion and the second portion, respectively, with the matrix of the oral care composition. The first portion of the oral care composition and the second portion of the oral care composition may be maintained separate from one another outside of the vessel.

The following numbered paragraphs disclose one or more exemplary variations of the subject matter of the application:
1. A method for preparing an oral care composition having a unique, multicolored pattern, the method comprising: forming a matrix in a base oral care composition; contacting a first colorant with a first portion of the base oral care composition; contacting a second colorant with a second portion of the base oral care composition; disposing the first portion of the oral care composition into a vessel via a first nozzle; disposing the second portion of the oral care composition into the vessel via a second nozzle; and forming a unique, multicolored pattern of the first and second colorants in the vessel.
2. The method of paragraph 1, wherein forming the unique, multicolored pattern of the first and second colorants in the vessel comprises one or more of: controlling a rate or volume of disposing the first portion into the vessel via the first nozzle; controlling a rate or volume of disposing the second portion into the vessel via the second nozzle; controlling a rate or volume ratio between disposing the first portion into the vessel via the first nozzle and disposing the second portion into the vessel via the second nozzle; or a combination thereof.
3. The method of paragraph 1 or 2, wherein forming the unique, multicolored pattern of the first and second colorants in the vessel comprises agitating the vessel while disposing the first portion of the oral care composition into the vessel, while disposing the second portion of the oral care composition into the vessel, or while disposing both the first and second portions of the oral care composition into the vessel.
4. The method of paragraph 3, wherein agitating the vessel comprises swirling the vessel.
5. The method of any one of paragraphs 1 to 4, further comprises disposing the first portion and the second portion into the vessel at the same time.
6. The method of any one of paragraphs 1 to 5, further comprising maintaining the unique, multicolored pattern of the first and second colorants in the vessel.
7. The method of paragraph 6, wherein maintaining the unique, multicolored pattern of the first and second colorants in the vessel comprises utilizing a pump bottle as the vessel.
8. The method of paragraph 6 or 7, wherein maintaining the unique, multicolored pattern of the first and second colorants in the vessel comprises forming the matrix in the base oral care composition, wherein the matrix is configured to inhibit or prevent the first colorant in the first portion of the oral care composition from diffusing into the second portion of the oral care composition.
9. The method of any one of paragraphs 1 to 9, wherein forming the matrix comprises contacting a combination of a thickening agent, an abrasive, an orally acceptable vehicle or a component thereof, or a combination thereof with one another.
10. The method of paragraph 9, wherein the thickening agent comprises a thickener silica, xanthan gum, or a combination thereof.
11. The method of paragraph 9 or 10, wherein the abrasive comprises a silica abrasive.
12. The method of any one of paragraphs 9 to 11, wherein the orally acceptable vehicle comprises a humectant and a surfactant, optionally, the humectant comprises glycerin, polyethylene glycol, or a combination thereof, further optionally, the surfactant comprises sodium lauryl sulfate.
13. The method of any one of paragraphs 9 to 12, wherein forming the matrix comprises contacting a combination of silica abrasive, a thickener silica, xanthan gum, glycerin, polyethylene glycol, and sodium lauryl sulfate with one another.
14. The method of any one of paragraphs 10 to 13, wherein the thickener silica is present in an amount of from about 2 wt% to about 8 wt%, about 3 wt% to about 7 wt%, about 4 wt% to about 6 wt%, or about 5 wt%, based on the total weight of the oral care composition.
15. The method of any one of paragraphs 11 to 14, wherein the silica abrasive is present in an amount of from about 6 wt% to about 10 wt%, about 7 wt% to about 9 wt%, or about 8 wt%, based on the total weight of the oral care composition.
16. The method of any one of paragraphs 11 to 15, wherein the abrasive silica and the thickener silica are present in a weight ratio of from about 1:1 to about 2:1, about 1.2:1 to about 1.8:1, about 1.4:1 to about 1.7:1, about 1.5:1 to about 1.6:1, or about 1.6:1.
17. The method of any one of paragraphs 10 to 16, wherein the xanthan gum is present in an amount of from greater than 0 wt% to about 2 wt%, about 0.2 wt% to about 1 wt%, about 0.3 wt% to about 0.7 wt%, or about 0.4 wt% to about 0.6 wt%, based on the total weight of the oral care composition.
18. The method of any one of paragraphs 12 to 17, wherein the glycerin is present in an amount of from about 45 wt% to about 65 wt%, about 50 wt% to about 60 wt%, about 55 wt% to about 57 wt%, or about 56 wt%, based on the total weight of the oral care composition.
19. The method of any one of paragraphs 12 to 18, wherein the polyethylene glycol is present in an amount of from about 1 wt% to about 5 wt%, about 2 wt% to about 4 wt%, or about 3 wt%, based on the total weight of the oral care composition.
20. The method of any one of paragraphs 12 to 19, wherein the sodium lauryl sulfate is present in an amount of from greater than 0 wt% to about 4 wt%, about 1 wt% to about 3 wt%, or about 2 wt%, based on the total weight of the oral care composition.
21. The method of any one of paragraphs 1 to 20, further comprising maintaining the unique, multicolored pattern of the first and second colorants in the vessel while dispensing the oral care composition from the vessel.
22. A method for enhancing aesthetic effects of an oral care composition, the method comprising the method for preparing the oral care composition of any one of paragraphs 1 to 21.
23. An oral care composition, comprising a base oral care composition comprising a matrix, the matrix being configured to prevent or inhibit a first colorant in a first portion of the base oral care composition from diffusing into or mixing with a second portion of the base oral care composition, the matrix comprising a thickening agent, an abrasive, an orally acceptable vehicle or a component thereof, or a combination thereof.
24. The oral care composition of paragraph 23, wherein: the thickening agent comprises a thickener silica, xanthan gum, or a combination thereof; the abrasive comprises a silica abrasive; the orally acceptable vehicle comprises a humectant and a surfactant, optionally, the humectant comprises glycerin, polyethylene glycol, or a combination thereof, further optionally, the surfactant comprises sodium lauryl sulfate.
25. The oral care composition of paragraphs 23 or 24, where the matrix comprises a combination of the silica abrasive, the thickener silica, xanthan gum, glycerin, polyethylene glycol, and sodium lauryl sulfate.

All ingredients for use in the compositions described herein should be orally acceptable. As used herein, "orally acceptable" may refer any ingredient that is present in a composition as described in an amount and form which does not render the composition unsafe for use in the oral cavity.

### EXAMPLES

The examples and other implementations described herein are exemplary and not intended to be limiting in describing the full scope of compositions and methods of this disclosure. Equivalent changes, modifications and variations of specific implementations, materials, compositions and methods may be made within the scope of the present disclosure, with substantially similar results.

### Example 1

Exemplary oral care compositions having a multicolored pattern were prepared by preparing a base composition without any colorants, according to Table 1. A first portion of the base composition was combined with a first water immiscible pigment and a second portion of the base composition was combined with a second water immiscible pigment.

**Table 1**

| **Base Composition** | |
|---|---|
| **Ingredient/Compo nent** | **Base Composition (wt%)** |
| Demineralized Water | 21.53 |
| Abrasive Silica | 8.00 |
| Thickener silica | 5.00 |
| Xanthan Gum | 0.40 |
| Glycerin | 56.00 |
| Polyethylene Glycol | 3.00 |
| Surfactant | 2.00 |
| Excipients | Balance |
| **Total Components** | **100** |

Each of the first and second portions of the base composition including their respective pigment was maintained separately until they were charged or disposed into a vessel (i.e., bottle). The first portion of the base composition including the first pigment was disposed into the vessel via a first nozzle, and the second portion of the base composition including the second pigment was disposed into the vessel via a second nozzle. It was observed that the combination of the abrasive silica, the thickener silica, the xanthan gum, glycerin, polyethylene glycol, and the surfactant, facilitated the formation of a matrix to keep the respective colorant/pigment in one portion from bleeding or blending into another portion of the oral care composition. The first and second portions were charged or disposed into the vessel at the same time while gently agitating or swirling the bottle. It was surprisingly and unexpectedly discovered that oral care compositions having unique and random multicolored patterns were able to be obtained by varying the ratio of each of the filling nozzles and the speed of agitating/swirling the bottle. It was further surprisingly and unexpectedly discovered that the general appearance or aesthetic effect of the multicolored pattern may be consistently provided in each sample (e.g., bottle) by controlling or maintaining the rate of dispensing the first portion into the vessel, the rate of dispensing the second portion into the vessel, and the rate of agitating the vessel while dispensing the portions into the vessel. It was also surprisingly and unexpectedly observed that the unique and random multicolored pattern formed in the oral care compositions were maintained with no color bleeding or diffusion observed. It was further surprisingly and unexpectedly discovered that the unique and random multicolored pattern formed in the oral care compositions upon manufacture were maintained with no color bleeding or diffusion under accelerated aging conditions, including stressed, relatively high temperature conditions. It was surprisingly and unexpectedly observed that the combination of the abrasive silica, the thickener silica, the xanthan gum, glycerin, polyethylene glycol, and the surfactant, facilitated the formation of a matrix to keep the respective colorant/pigment in one portion from bleeding or blending into another portion of the oral care composition.

It was observed that utilizing a pump to dispense the oral care composition allowed the composition and the matrix thereof to maintain the unique and random multicolored pattern within the bottle while concurrently delivering a plurality of colors through the nozzle of the pump. Particularly, the unique and random multicolored pattern formed in the bottle was maintained and merely shifted down as the pump dispensed the oral care composition from the bottom of the bottle. Conversely, it was observed that utilizing a collapsible tube altered or destroyed the unique multicolored pattern formed upon manufacture and/or result in the bleeding, blending, or diffusion of the colorant from one portion of the oral care composition with the colorant from another portion of the oral care composition.

The present disclosure has been described with reference to exemplary implementations. Although a limited number of implementations have been shown and described, it will be appreciated by those skilled in the art that changes may be made in these implementations without departing from the principles and spirit of the preceding detailed description. It is intended that the present disclosure be construed as including all such modifications and alterations insofar as they come within the scope of the appended claims or the equivalents thereof.
The invention also refers to the following numbered embodiments, wherein the term "claim" refers to "emmbodiments".
1. A method for preparing an oral care composition having a unique, multicolored pattern, the method comprising:
   forming a matrix in a base oral care composition;
   contacting a first colorant with a first portion of the base oral care composition;
   contacting a second colorant with a second portion of the base oral care composition;
   disposing the first portion of the oral care composition into a vessel via a first nozzle;
   disposing the second portion of the oral care composition into the vessel via a second nozzle; and
   forming a unique, multicolored pattern of the first and second colorants in the vessel.
2. The method of claim 1, wherein forming the unique, multicolored pattern of the first and second colorants in the vessel comprises one or more of:
   controlling a rate or volume of disposing the first portion into the vessel via the first nozzle;
   controlling a rate or volume of disposing the second portion into the vessel via the second nozzle;
   controlling a rate or volume ratio between disposing the first portion into the vessel via the first nozzle and disposing the second portion into the vessel via the second nozzle;
   or a combination thereof.
3. The method of claim 1 or 2, wherein forming the unique, multicolored pattern of the first and second colorants in the vessel comprises agitating the vessel while disposing the first portion of the oral care composition into the vessel, while disposing the second portion of the oral care composition into the vessel, or while disposing both the first and second portions of the oral care composition into the vessel.
4. The method of claim 3, wherein agitating the vessel comprises swirling the vessel.
5. The method of any one of the foregoing claims, further comprises disposing the first portion and the second portion into the vessel at the same time.
6. The method of any one of the foregoing claims, further comprising maintaining the unique, multicolored pattern of the first and second colorants in the vessel.
7. The method of claim 6, wherein maintaining the unique, multicolored pattern of the first and second colorants in the vessel comprises utilizing a pump bottle as the vessel.
8. The method of claim 6 or 7, wherein maintaining the unique, multicolored pattern of the first and second colorants in the vessel comprises forming the matrix in the base oral care composition, wherein the matrix is configured to inhibit or prevent the first colorant in the first portion of the oral care composition from diffusing into the second portion of the oral care composition.
9. The method of any one of the foregoing claims, wherein forming the matrix comprises contacting a combination of a thickening agent, an abrasive, an orally acceptable vehicle or a component thereof, or a combination thereof with one another.
10. The method of claim 9, wherein the thickening agent comprises a thickener silica, xanthan gum, or a combination thereof.
11. The method of claim 9 or 10, wherein the abrasive comprises a silica abrasive.
12. The method of any one of claims 9 to 11, wherein the orally acceptable vehicle comprises a humectant and a surfactant, optionally, the humectant comprises glycerin, polyethylene glycol, or a combination thereof, further optionally, the surfactant comprises sodium lauryl sulfate.
13. The method of any one of claims 9 to 12, wherein forming the matrix comprises contacting a combination of silica abrasive, a thickener silica, xanthan gum, glycerin, polyethylene glycol, and sodium lauryl sulfate with one another.
14. The method of any one of claims 10 to 13, wherein the thickener silica is present in an amount of from about 2 wt% to about 8 wt%, about 3 wt% to about 7 wt%, about 4 wt% to about 6 wt%, or about 5 wt%, based on the total weight of the oral care composition.
15. The method of any one of claims 11 to 14, wherein the silica abrasive is present in an amount of from about 6 wt% to about 10 wt%, about 7 wt% to about 9 wt%, or about 8 wt%, based on the total weight of the oral care composition.
16. The method of any one of claims 11 to 15, wherein the abrasive silica and the thickener silica are present in a weight ratio of from about 1:1 to about 2:1, about 1.2:1 to about 1.8:1, about 1.4:1 to about 1.7:1, about 1.5:1 to about 1.6:1, or about 1.6:1.
17. The method of any one of claims 10 to 16, wherein the xanthan gum is present in an amount of from greater than 0 wt% to about 2 wt%, about 0.2 wt% to about 1 wt%, about 0.3 wt% to about 0.7 wt%, or about 0.4 wt% to about 0.6 wt%, based on the total weight of the oral care composition.
18. The method of any one of claims 12 to 17, wherein the glycerin is present in an amount of from about 45 wt% to about 65 wt%, about 50 wt% to about 60 wt%, about 55 wt% to about 57 wt%, or about 56 wt%, based on the total weight of the oral care composition.
19. The method of any one of claims 12 to 18, wherein the polyethylene glycol is present in an amount of from about 1 wt% to about 5 wt%, about 2 wt% to about 4 wt%, or about 3 wt%, based on the total weight of the oral care composition.
20. The method of any one of claims 12 to 19, wherein the sodium lauryl sulfate is present in an amount of from greater than 0 wt% to about 4 wt%, about 1 wt% to about 3 wt%, or about 2 wt%, based on the total weight of the oral care composition.
21. The method of any one of the foregoing claims, further comprising maintaining the unique, multicolored pattern of the first and second colorants in the vessel while dispensing the oral care composition from the vessel.
22. A method for enhancing aesthetic effects of an oral care composition, the method comprising the method for preparing the oral care composition of any one of the foregoing claims.
23. An oral care composition, comprising a base oral care composition comprising a matrix, the matrix being configured to prevent or inhibit a first colorant in a first portion of the base oral care composition from diffusing into or mixing with a second portion of the base oral care composition, the matrix comprising a thickening agent, an abrasive, an orally acceptable vehicle or a component thereof, or a combination thereof.
24. The oral care composition of claim 23, wherein:
   the thickening agent comprises a thickener silica, xanthan gum, or a combination thereof;
   the abrasive comprises a silica abrasive;
   the orally acceptable vehicle comprises a humectant and a surfactant, optionally, the humectant comprises glycerin, polyethylene glycol, or a combination thereof, further optionally, the surfactant comprises sodium lauryl sulfate.
25. The oral care composition of claim 23 or 24, where the matrix comprises a combination of the silica abrasive, the thickener silica, xanthan gum, glycerin, polyethylene glycol, and sodium lauryl sulfate.

## Claims

1. A method for preparing an oral care composition having a unique, multicolored pattern, the method comprising:
forming a matrix in a base oral care composition;
contacting a first colorant with a first portion of the base oral care composition;
contacting a second colorant with a second portion of the base oral care composition;
disposing the first portion of the oral care composition into a vessel via a first nozzle;
disposing the second portion of the oral care composition into the vessel via a second nozzle;
optionally disposing the first portion and the second portion into the vessel at the same time;
and
forming a unique, multicolored pattern of the first and second colorants in the vessel.

2. The method of claim 1, wherein forming the unique, multicolored pattern of the first and second colorants in the vessel comprises one or more of:
controlling a rate or volume of disposing the first portion into the vessel via the first nozzle;
controlling a rate or volume of disposing the second portion into the vessel via the second nozzle;
controlling a rate or volume ratio between disposing the first portion into the vessel via the first nozzle and disposing the second portion into the vessel via the second nozzle;
or a combination thereof.

3. The method of claim 1 or 2, wherein forming the unique, multicolored pattern of the first and second colorants in the vessel comprises agitating the vessel while disposing the first portion of the oral care composition into the vessel, while disposing the second portion of the oral care composition into the vessel, or while disposing both the first and second portions of the oral care composition into the vessel, wherein agitating the vessel optionally comprises swirling the vessel.

4. The method of any one of the foregoing claims, further comprising maintaining the unique, multicolored pattern of the first and second colorants in the vessel.

5. The method of claim 4, wherein maintaining the unique, multicolored pattern of the first and second colorants in the vessel comprises utilizing a pump bottle as the vessel.

6. The method of claim 4 or 5, wherein maintaining the unique, multicolored pattern of the first and second colorants in the vessel comprises forming the matrix in the base oral care composition, wherein the matrix is configured to inhibit or prevent the first colorant in the first portion of the oral care composition from diffusing into the second portion of the oral care composition.

7. The method of any one of the foregoing claims, wherein forming the matrix comprises contacting a combination of a thickening agent (such as a thickener silica, xanthan gum, or a combination thereof), an abrasive (such as a silica abrasive), an orally acceptable vehicle or a component thereof, or a combination thereof with one another.

8. The method of claim 7, wherein the orally acceptable vehicle comprises a humectant and a surfactant, optionally, the humectant comprises glycerin, polyethylene glycol, or a combination thereof, further optionally, the surfactant comprises sodium lauryl sulfate.

9. The method of claim 7 or 8, wherein forming the matrix comprises contacting a combination of silica abrasive, a thickener silica, xanthan gum, glycerin, polyethylene glycol, and sodium lauryl sulfate with one another.

10. The method of claim 8 or 9, wherein the thickener silica is present in an amount of from about 2 wt% to about 8 wt%, about 3 wt% to about 7 wt%, about 4 wt% to about 6 wt%, or about 5 wt%, based on the total weight of the oral care composition; and/or
wherein the silica abrasive is present in an amount of from about 6 wt% to about 10 wt%, about 7 wt% to about 9 wt%, or about 8 wt%, based on the total weight of the oral care composition; and/or
wherein the xanthan gum is present in an amount of from greater than 0 wt% to about 2 wt%, about 0.2 wt% to about 1 wt%, about 0.3 wt% to about 0.7 wt%, or about 0.4 wt% to about 0.6 wt%, based on the total weight of the oral care composition; and/or
wherein the glycerin is present in an amount of from about 45 wt% to about 65 wt%, about 50 wt% to about 60 wt%, about 55 wt% to about 57 wt%, or about 56 wt%, based on the total weight of the oral care composition; and/or
wherein the polyethylene glycol is present in an amount of from about 1 wt% to about 5 wt%, about 2 wt% to about 4 wt%, or about 3 wt%, based on the total weight of the oral care composition; and/or
wherein the sodium lauryl sulfate is present in an amount of from greater than 0 wt% to about 4 wt%, about 1 wt% to about 3 wt%, or about 2 wt%, based on the total weight of the oral care composition.

11. The method of any one of claims 8 to 10, wherein the abrasive silica and the thickener silica are present in a weight ratio of from about 1:1 to about 2:1, about 1.2:1 to about 1.8:1, about 1.4:1 to about 1.7:1, about 1.5:1 to about 1.6:1, or about 1.6:1.

12. The method of any one of the foregoing claims, further comprising maintaining the unique, multicolored pattern of the first and second colorants in the vessel while dispensing the oral care composition from the vessel.

13. A method for enhancing aesthetic effects of an oral care composition, the method comprising the method for preparing the oral care composition of any one of the foregoing claims.

14. An oral care composition, comprising a base oral care composition comprising a matrix, the matrix being configured to prevent or inhibit a first colorant in a first portion of the base oral care composition from diffusing into or mixing with a second portion of the base oral care composition, the matrix comprising a thickening agent, an abrasive, an orally acceptable vehicle or a component thereof, or a combination thereof.

15. The oral care composition of claim 14, wherein:
the thickening agent comprises a thickener silica, xanthan gum, or a combination thereof;
the abrasive comprises a silica abrasive;
the orally acceptable vehicle comprises a humectant and a surfactant, optionally, the humectant comprises glycerin, polyethylene glycol, or a combination thereof, further optionally, the surfactant comprises sodium lauryl sulfate,
optionally wherein the matrix comprises a combination of the silica abrasive, the thickener silica, xanthan gum, glycerin, polyethylene glycol, and sodium lauryl sulfate.
